(19) Europäisches Patentamt
European Patent Office
Office européen des brevets

(11) Veröffentlichungsnummer: **0 042 103**
**A1**

(12) **EUROPÄISCHE PATENTANMELDUNG**

(21) Anmeldenummer: **81104258.9**

(22) Anmeldetag: **03.06.81**

(51) Int. Cl.³: **C 07 D 491/147**, A 61 K 31/44 // (C07D491/147, 209/00, 307/00)

(30) Priorität: **14.06.80 DE 3022357**

(43) Veröffentlichungstag der Anmeldung: **23.12.81 Patentblatt 81/51**

(84) Benannte Vertragsstaaten: **AT BE CH DE FR GB IT LI LU NL SE**

(71) Anmelder: **Troponwerke GmbH & Co. KG, Berliner Strasse 156, D-5000 Köln 80 (DE)**

(72) Erfinder: **Boltze, Karl-Heinz, Dr., Am Burgtor 23, D-5060 Berg.-Gladbach 3 (DE)**
Erfinder: **Seidel, Peter-Rudolf, Dr., Alte Heide 5d, D-5000 Koeln 90 (DE)**
Erfinder: **Jacobi, Haireddin, Dr., Finkenweg 2, D-5653 Leichlingen (DE)**
Erfinder: **Schwarz, Helmut, Dr., Am Kastanienberg 17, D-6903 Neckargemünd (DE)**
Erfinder: **Schöllnhammer, Günter, Dr., Hackberg 21, D-5060 Bergisch-Gladbach (DE)**

(74) Vertreter: **Adrian, Albert, Dr. et al, BAYER AG c/o Zentralbereich Patente Marken und Lizenzen, D-5090 Leverkusen 1, Bayerwerk (DE)**

(54) **7,8,9,10-Tetrahydrofuro(3,2-e)pyrido(4,3-b)indole, Verfahren zu ihrer Herstellung und ihre Verwendung in Arzneimitteln sowie deren Herstellung.**

(57) Die vorliegende Erfindung betrifft neue 7,8,9,10-Tetrahydrofuro[3,2-e]pyrido[4,3-b]indole, ein Verfahren zu ihrer Herstellung sowie ihre Verwendung in Arzneimitteln, insbesondere in Arzneimitteln, die das zentrale Nervensystem beeinflussen.

EP 0 042 103 A1

TROPONWERKE GmbH & Co KG

KS/bc/c
Ia (Pha)

7,8,9,10-Tetrahydrofuro[3,2-e]pyrido[4,3-b]indole, Verfahren zu ihrer Herstellung und ihre Verwendung in Arzneimitteln sowie deren Herstellung

Die vorliegende Erfindung betrifft neue 7,8,9,10-Tetrahydrofuro[3,2-e]pyrido[4,3-b]indole, ein Verfahren zu ihrer Herstellung sowie ihre Verwendung in Arzneimitteln, insbesondere in Arzneimitteln, die das zentrale Nervensystem beeinflussen.

In der Literatur sind bereits Verfahren zur Herstellung von Indolderivaten mit ankondensierten heterocyclischen Ringen beschrieben. Eine therapeutische Anwendbarkeit ist für diese Verbindungen bisher nicht bekannt geworden (vgl. N.M. Sharkova et al., Khim. Geterotsikl. Soedin. 1972, 1075-1078, zitiert in C.A. Vol. 77, 152028 t, 408-409 (1972)). Für keine dieser bekannten Indolverbindungen mit ähnlicher chemischer Struktur konnte eine Wirkung auf das zentrale Nervensystem gefunden werden, die ihre Verwendung in Arzneimitteln nahegelegt hätten.

Die Erfindung betrifft neue 7,8,9,10-Tetrahydrofuro-[3,2-e]pyrido[4,3-b]indol-Derivate der allgemeinen Formel (I)

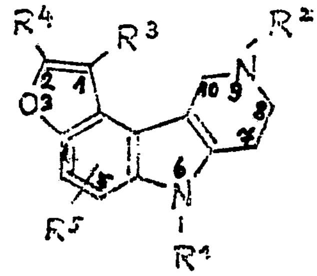

(I)

in welcher

$R^1$ und $R^2$ gleich oder verschieden sind und jeweils für Wasserstoff, geradkettiges, verzweigtes oder cyclisches Alkyl, Alkenyl, Alkinyl, Aryl, Acyl, Aroyl, Mono- und Dialkylaminoalkyl, Arylalkylaminoalkyl, Heteroarylalkylaminoalkyl oder Acylamino stehen, wobei die genannten Alkyl-, Alkenyl- und Arylreste gegebenenfalls substituiert sind durch Halogen, Nitro, Amino, Alkyl, Trifluormethyl, Alkoxy, Hydroxy, Acylamino, gegebenenfalls substituiertes Phenyl, Heteroaryl oder durch einen 5-, 6- oder 7-gliedrigen gesättigten oder ungesättigten aliphatischen Ring, der 1 oder 2 gleiche oder verschiedene Heteroatome aus der Gruppe Sauerstoff, Schwefel oder N-R' enthalten kann, wobei

R' für Wasserstoff, Alkyl, Alkenyl, Aryl, Aralkyl, Acyl oder Aroyl steht,

$R^3$ und $R^4$ gleich oder verschieden sind und jeweils für Wasserstoff, geradkettiges, verzweigtes oder cyclisches Alkyl, Alkenyl oder Alkinyl, Aryl, Acyl oder Aroyl stehen, wobei die genannten Alkyl-, Alkenyl- und Arylreste gegebenenfalls substituiert sind

durch Halogen, Alkyl, Alkoxy, Acylamino, gegebenenfalls substituiertes Phenyl oder Heteroaryl, wobei $R^3$ nicht für Wasserstoff steht, wenn $R^2$ und $R^4$ Methyl bedeuten und $R^1$ Wasserstoff bedeutet, oder

$R^3$ und $R^4$ gemeinsam für einen gegebenenfalls verzweigten Alkylenrest stehen, durch Sauerstoff oder durch die Gruppe $S(O)_n$ unterbrochen sein kann, wobei n für 0, 1 oder 2 steht,

$R^5$ für Wasserstoff, Halogen, Alkyl, Alkoxy oder Phenyl steht,

als solche, in Form ihrer quarternären Ammoniumsalze gebildet mit Alkylhalogeniden und ihre pharmakologisch unbedenklichen Säureadditionssalze.

Die vorliegende Erfindung betrifft weiterhin ein Verfahren zur Herstellung von Verbindungen der allgemeinen Formel (I), dadurch gekennzeichnet, daß man Hydrazinverbindungen der allgemeinen Formel (II)

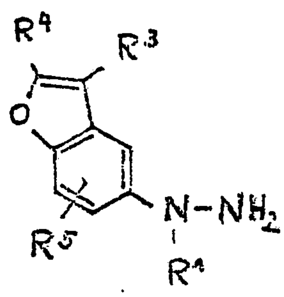

(II)

in welcher

$R^1$,$R^3$,$R^4$ und $R^5$ die oben angegebene Bedeutung haben,

mit Piperidonen der allgemeinen Formel (III)

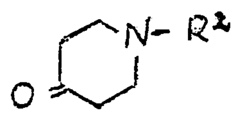

(III)

in welcher
$R^2$ die oben angegebene Bedeutung hat,

bzw. mit Salzen dieser Piperidone mit anorganischen oder organischen Säuren in Gegenwart von inerten organischen Lösungsmitteln bei Temperaturen zwischen 20 und 250°C, gegebenenfalls in Gegenwart von Kondensationsmitteln, umsetzt und die so erhaltenen Verbindungen der Formel (I) gegebenenfalls anschließend in bekannter Weise in die quaternären Ammoniumsalze oder die Säureadditionssalze überführt und für den Fall, daß die Stickstoffsubstituenten $R^1$ und $R^2$ Wasserstoff bedeuten, diese nach bekannten Methoden durch andere erfindungsgemäße Substituenten ersetzt.

Die neuen Verbindungen der allgemeinen Formel (I) zeigen überraschenderweise ausgeprägte und vorteilhafte Wirkungen auf das zentrale Nervensystem. Besonders erwähnt sei ihre Verwendung als Antidepressiva. Die antidepressive Wirkung der Verbindungsgruppe konnte bei Kenntnis des Standes der Technik nicht erwartet werden. Da erfahrungsgemäß verschiedene Verbindungsgruppen im Körper unterschiedliche Metabolisierungen durchlaufen und somit für diese Verbindungen auch ein neuer Wirkungsmechanismus anzunehmen ist, stellen sie eine Bereicherung der Pharmazie dar. Sie können auch in solchen Fällen eingesetzt werden, in denen bekannte

Antidepressiva Unverträglichkeiten oder Gewöhnung hervorrufen.

Als eine interessante pharmakologische Wirkung sei die Amphetamin-potenzierende Wirkung der erfindungsgemäßen Verbindungen genannt. Diese Amphetamin-potenzierende Wirkung wurde bestimmt nach der Methode von Boksay et al., Arzneimittelforschung 29, 193 (1979). Es zeigt sich, daß die erfindungsgemäßen Verbindungen bei peroraler Applikation bereits bei Dosierungen unter 20 mg/kg eine signifikante Amphetamin-potenzierende Wirkung besitzen. Sie können somit als Phsychopharmaka, insbesondere als Antidepressiva, eingesetzt werden, zumal sie gleichzeitig eine gute Verträglichkeit besitzen.

Neben der Amphetamin-potenzierenden Wirkung besitzen die erfindungsgemäßen Verbindungen noch weitere Eigenschaften, die auf ein breites und vielseitiges pharmakologisches Wirkungsspektrum hinweisen. Im einzelnen seien folgende Hauptwirkungen genannt: Die Verbindungen besitzen eine antagonistische Wirkung gegenüber Reserpin, Butyrylperazin und Clopenthixol.

Die neuen Verbindungen besitzen analgetische und antiphlogistische Wirkungen,die z.B. am Kaolin-Pfotenödem bei Ratten nachgewiesen werden können (vgl.Jakobi et al., Arzneimittelforschung 27, 1926 (1977)).

Von besonderer Bedeutung sind Verbindungen der allgemeinen Formel (I) und ebenfalls die als Ausgangsverbindungen eingesetzten Verbindungen der Formeln

(II) und (III), in welchen

$R^1$ und $R^2$ gleich oder verschieden sind und jeweils für Wasserstoff, geradkettiges, verzweigtes oder cyclisches Alkyl, Alkenyl oder Alkinyl mit bis zu 12 Kohlenstoffatomen, Aralkyl mit 7 bis 16 Kohlenstoffatomen, Phenyl, Acyl mit bis zu 8 Kohlenstoffatomen, Benzoyl, Mono- und Dialkylaminoalkyl und Phenylalkylaminoalkyl mit jeweils bis zu 4 Kohlenstoffatomen in den Alkylresten, oder Acylamino. dessen Stickstoffatom substituiert sein kann durch 1 oder 2 Alkylreste mit 1 bis 4 Kohlenstoffatomen oder durch Phenyl, wobei die beiden Alkylgruppen gegebenenfalls mit dem Stickstoffatom einen 5- bis 7-gliedrigen Ring bilden, der gegebenenfalls ein weiteres Heteroatom aus der Gruppe Sauerstoff, NH oder N-Alkyl mit 1-4 Kohlenstoffatomen enthält, stehen, wobei die vorgenannten Alkyl-, Alkenyl- und Phenylreste ihrerseits gegebenenfalls substituiert sind durch Halogen, Nitro, Amino, Hydroxy, Alkyl mit 1 bis 4 Kohlenstoffatomen, Alkoxy mit 1 bis 4 Kohlenstoffatomen, Acylamino mit 1 bis 4 Kohlenstoffatomen in der Acylgruppe, Phenyl, Pyridyl oder durch einen 5-, 6- oder 7-gliedrigen gesättigten oder ungesättigten aliphatischen Ring, der 1 oder 2 gleiche oder verschiedene Heteroatome aus der Gruppe Sauerstoff, Schwefel oder NR' enthalten kann, wobei

R' für Wasserstoff, Alkyl mit 1 bis 4 Kohlenstoffatomen, Phenyl, Benzyl oder Acyl mit bis zu 4 Kohlenstoffatomen steht,

$R^3$ und $R^4$ gleich oder verschieden sind und jeweils für Wasserstoff, geradkettiges, verzweigtes oder cyclisches Alkyl, Alkenyl oder Alkinyl mit bis zu 12 Kohlenstoffatomen, Aryl mit 6 bis 12 Kohlenstoffatomen, Acyl mit bis zu 4 Kohlenstoffatomen oder

Benzoyl stehen, wobei die vorgenannten Alkyl-, Alkenyl- und Arylreste gegebenenfalls substituiert sind durch Halogen, Trifluormethyl, Alkyl mit 1 bis 4 Kohlenstoffatomen, Alkoxy mit 1 bis 4 Kohlenstoffatomen, Acylamino mit bis zu 4 Kohlenstoffatomen, Phenyl oder Pyridyl, wobei der Phenylrest gegebenenfalls 1 oder 2 gleiche oder verschiedene Substituenten aus der Gruppe Halogen, Trifluormethyl, Alkyl oder Alkoxy mit jeweils 1 bis 2 Kohlenstoffatomen substituiert ist,

wobei $R^3$ nicht Wasserstoff bedeutet, wenn $R^2$ und $R^4$ Methyl bedeuten und $R^1$ Wasserstoff bedeutet,
oder

$R^3$ und $R^4$ gemeinsam für einen gegebenenfalls verzweigten Alkylenrest mit bis zu 10 Kohlenstoffatomen stehen, der durch Sauerstoff, Schwefel oder die Gruppe $SO_2$ unterbrochen sein kann, und

$R^5$ für Wasserstoff, Halogen, Phenyl, Alkyl oder Alkoxy mit je 1 bis 4 Kohlenstoffatomen steht.

Von besonderer Bedeutung sind Verbindungen der allgemeinen Formel (I), in welcher

$R^1$ und $R^2$ gleich oder verschieden sind und jeweils für Wasserstoff, geradkettiges, verzweigtes oder cyclisches Alkyl oder Alkenyl mit bis zu 8 Kohlenstoffatomen, Phenyl, Acyl mit bis zu 4 Kohlenstoffatomen, Benzoyl oder Acylamino stehen, wobei der Stickstoff des Aminorestes gegebenenfalls durch 1 oder 2 Alkylgruppen substituiert ist oder ein Ringglied eines Morpholin-, Piperidin- oder Piperazinrestes darstellt, deren Ringe ihrerseits gegebenenfalls durch Methyl oder

Ethyl substituiert sind, wobei die vorgenannten Alkyl-, Alkenyl-, Phenyl- und Acylreste ihrerseits gegebenenfalls substituiert sind durch Alkyl mit 1 bis 4 Kohlenstoffatomen, Mono- oder Dialkylamino mit jeweils 1 bis 4 Kohlenstoffatomen im Alkylrest, Halogen, Nitro, Amino, Trifluormethyl, Trifluormethoxy, Alkoxy mit 1 bis 2 Kohlenstoffatomen im Alkoxyrest, Phenyl, Piperidin, Acetylamino, Morpholino oder Cyano,

$R^3$ und $R^4$ gleich oder verschieden sind und jeweils für Wasserstoff, Alkyl mit 1 bis 4 Kohlenstoffatomen, Phenyl oder Trifluormethyl stehen, wobei die genannten Phenyl- und Alkylreste ihrerseits gegebenenfalls substituiert sind durch Halogen, Alkyl oder Alkoxy mit jeweils 1 bis 2 Kohlenstoffatomen, wobei $R^3$ nicht für Wasserstoff steht, wenn $R^2$ und $R^4$ Methyl bedeuten und $R^1$ Wasserstoff bedeutet,

oder

$R^3$ und $R^4$ gemeinsam für einen gegebenenfalls verzweigten Alkylenrest mit bis zu 6 Kohlenstoffatomen stehen, der durch Sauerstoff oder Schwefel in der Kette unterbrochen sein kann und

$R^5$ für Wasserstoff, Halogen oder Methyl steht.

Bei der Herstellung der erfindungsgemäßen Verbindungen der allgemeinen Formel (I) aus Hydrazinverbindungen der allgemeinen Formel (II) und Piperidonen der allgemeinen Formel (III) kann je nach Reaktivität der Reaktionspartner nach zwei verschiedenen Varianten verfahren werden:

a) Bei den säureempfindlichen Reaktionspartnern (II und III) setzt man deren Salze, vorzugsweise die Hydrochloride, in einem geeigneten Verdünnungsmittel ein.

b) Bei Reaktionspartnern (II und III), die sich bevorzugt in Form ihrer Basen zu Verbindungen der Formel (I) umsetzen oder die sich durch Säureunempfindlichkeit auszeichnen, arbeitet man vorzugsweise bei höheren Temperaturen in Gegenwart von hochsiedenden Lösungsmitteln oder ohne Lösungsmittel.

Bei beiden Reaktionsvarianten ist es vorteilhaft, die Reaktion unter Inertgasatmosphäre, wie z.B. Stickstoff oder Argon, durchzuführen.

Als geeignete Lösungsmittel für die Verfahrensvariante a) eignen sich alle Lösungsmittel, die für die Fischer-Indol-Synthese üblich sind (vgl. E. Enders, Houben-Weyl, Band 10/2, S. 546-586 (1967); A. Weissberger, The Chemistry of Heterocyclic Compounds, Indole, Part I, S. 232-370 (1972)).

Beispielhaft seien genannt: Wasser, Methanol, Ethanol, Propyl-, Isopropylalkohol, Benzol, Toluol, Xylol, Dioxan, Eisessig, Propionsäure, Polyphosphorsäureethylester oder hochsiedende Kohlenwasserstoffe.

Als Kondensationsmittel werden ebenfalls die für den Indolringschluß üblichen Katalysatoren verwendet. Beispielhaft seien genannt: Zinkchlorid, Bortrifluorid, Bortrifluoridetherat, Chlorwasserstoff, Salzsäure, Schwefelsäure, Phosphorsäure, Polyphosphorsäure, Polyphosphorsäureethylester, Ameisensäure, Trifluoressigsäure, saure Ionenaustauscher wie z.B. Amberlite oder ein Gemisch aus Eisessig-Chlorwasserstoff.

Die Reaktionstemperaturen können in einem größeren Bereich variiert werden. Im allgemeinen arbeitet man zwischen 40°C und 150°C, vorzugsweise zwischen 60°C und 120°C. Die Reaktionszeiten bewegen sich zwischen 0,5 und 20 Stunden je nach Reaktionstemperatur.

Die Umsetzung wird normalerweise bei Atmosphärendruck durchgeführt.

Bei der Durchführung des erfindungsgemäßen Verfahrens (a) setzt man vorteilhaft auf ein Mol der Hydrazinverbindung (II) das Piperidon (III) in einem Überschuß von 0,1 bis 0,5 Mol ein.

Zweckmäßigerweise kann die Umsetzung auch unter Inertgas, wie z.B. Stickstoff oder Argon, durchgeführt werden. Das Keton der allgemeinen Formel (III) kann in einzelnen Fällen vorteilhaft auch als entsprechendes Ketal wie z.B. Ethylenketal oder Propylenketal eingesetzt werden.

Die Reaktionsvariante b) wird vorzugsweise durchgeführt bei Temperaturen zwischen 150 und 250°C, insbesondere in einem Bereich zwischen 180 und 210°C. Die Verwendung

von Inertgasatmosphäre ist vorteilhaft. Als Lösungsmittel für diese Reaktionsvariante seien beispielhaft genannt: Tetralin, Dichlorbenzol, Acetamid, Ethylenglykol, Diethylenglykol, Diethylenglykolmonomethylether, Triethylenglykol, Glycerin, N-Methyl-pyrrolidon, Ethylenglykoldimethylether, Diethylenglykoldibutylether, wobei Ethylenglykol besonders bevorzugt genannt sei.

Verbindungen der allgemeinen Formel (I), in denen die Substituenten $R^1$ und/oder $R^2$ am Stickstoff für Wasserstoff stehen, lassen sich nach bekannten Methoden nachträglich in entsprechend substituierte Verbindungen überführen. Eine solche nachträgliche Substitution erfolgt vorzugsweise, indem man z.B. die NH-Verbindung mit Natrium, Kalium, Natriumamid, Kaliumamid, Kaliumhydroxid, Lithiumhydroxid, Natriumhydrid oder Alkalialkoholat, wie beispielsweise Natriumethylat oder Kaliummethylat, vorzugsweise mit Natriumhydrid, in das Alkalisalz (I, $R^1$ = Na, K oder Li) umwandelt und dieses in an sich bekannter Weise mit den entsprechend substituierten Halogeniden, wie z.B. Säurehalogenide aliphatischer, alicyclischer oder aromatischer Carbonsäuren oder mit den entsprechend substituierten aliphatischen, alicyclischen oder aromatischen Halogenverbindungen, die in ihren Resten unsubstituierte oder durch Alkyl, Aralkyl, Cycloalkyl oder Aryl mono- oder disubstituierte $NH_2$-Gruppen tragen können, umsetzt.

Die Aroylierung und Alkylierung der NH-Gruppen im Pyrrol- und Tetrahydropyridinteil von I mit $R^1$ und/oder $R^2$ für H kann gegebenenfalls bei ausreichender Basizität dieser Gruppen auch direkt in Gegenwart geeigneter Protonenakzeptoren wie Trimethylamin, Triethylamin, N-Methylmorpholin, N-Methylpiperidin, N,N-Dimethylanilin, N,N-Diethylanilin, heterocyclische Basen wie Pyridin, Picoline, Kollidine, Chinolin oder Isochinolin durchgeführt werden.

Die Umsetzung kann ohne Lösemittel oder aber in Gegenwart geeigneter Lösungsvermittler durchgeführt werden. Als Lösungsvermittler kommen alle organischen Lösemittel in Frage, die gegenüber den jeweiligen Reaktionspartnern inert sind. Hierzu gehören vorzugsweise aromatische Kohlenwasserstoffe wie Benzol, Toluol, Xylol oder Tetralin, Ether wie Diethylether, Diisopropylether, Tetrahydrofuran, Dioxan, Ethylenglykoldiethylether, Nitrile wie Acetonitril, Propionitril, Carbonsäureamide wie Dimethylformamid, Dimethylacetamid, Hexamethylphosphorsäuretriamid, N-Methylpyrrolidon, Dimethylsulfoxid, heterocyclische Basen wie Pyridin, Chinolin oder Picoline, ferner handelsübliche technische Gemische dieser Lösemittel.

Die Umsetzung kann bei Normaldruck aber auch bei erhöhtem Druck durchgeführt werden, insbesondere bei niedrig siedenden Alkylhalogeniden als Reaktionspartner kann erhöhter Druck für die Umsetzung erforderlich sein.

Die Reaktionstemperaturen können in einem gewissen Bereich variiert werden. Im allgemeinen arbeitet man bei Temperaturen zwischen 0 und 200°C, vorzugsweise zwischen 20 und 150°C, insbesondere zwischen 40 und 80°C; in einzelnen Fällen genügt Raumtemperatur.

Die Aufarbeitung erfolgt dann wieder analog wie oben beschrieben.

Die als Ausgangsstoffe zu verwendenden Hydrazine der Formel (II) sind bekannt oder lassen sich nach bekannten Verfahren beispielhaft nach folgendem Schema herstellen:

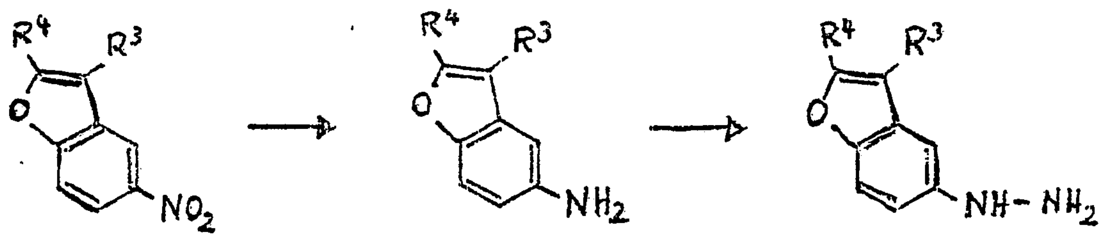

(II)

vgl. (a) T. Sheradsky, Tetrah. Letters 1966, 5225;
(b) A. Mustafa in "The Chemistry of Heterocyclic Compounds", Vol. 29: Benzofurans (Ed.: A. Weissberger und E.C. Taylor), S. 1-77, Wiley-Interscience, New York, 1974;
(c) P. Cagniant und D. Cagniant in "Advances in Heterocyclic Chemistry", Vol. 18: Recent Advances in the Chemistry of Benzo[b]furan and Its Derivatives, Part I (Ed.: A.R. Katritzky und A.J. Boulton), S. 337-482. Academic Press, New York, 1975;

(d) E. Enders in Houben-Weyl, Bd. 10/2: Methoden zur Herstellung und Umwandlung von Arylhydrazinen und Arylhydrazonen, S. 177-409 (1967).

Als neue Wirkstoffe seien im einzelnen genannt:

9-Methyl-6-(4-acetylaminophenyl)-6H-7,8,9,10-tetrahydrofuro[3,2-e] pyrido[4,3-b]indol;
1-Methyl-6H-7,8,9,10-tetrahydrofuro[3,2-e]pyrido[4,3-b]indol;
1-Methyl-9-ethyl-6H-7,8,9,10-tetrahydrofuro[3,2-e]pyrido[4,3-b]indol;
1-Methyl-9-benzyl-6H-7,8,9,10-tetrahydrofuro[3,2-e]pyrido[4,3-b]indol;
2-Methyl-6H-7,8,9,10-tetrahydrofuro[3,2-e]pyrido[4,3-b]indol;
2-Methyl-9-acetyl-6H-7,8,9,10-tetrahydrofuro[3,2-e]pyrido[4,3-b]indol;
2-Methyl-9-benzoyl-6H-7,8,9,10-tetrahydrofuro[3,2-e]pyrido[4,3-b]indol;
2-Methyl-9-diethylaminocarbonyl-6H-7,8,9,10-tetrahydrofuro[3,2-e]pyrido[4,3-b]indol;
2-Methyl-9-piperidinocarbonyl-6H-7,8,9,10-tetrahydrofuro[3,2-e]pyrido[4,3-b]indol;
2-Methyl-9-(4-methylpiperazinocarbonyl)-6H-7,8,9,10-tetrahydrofuro[3,2-e]pyrido[4,3-b]indol;
2,9-Dimethyl-6-ethyl-6H-7,8,9,10-tetrahydrofuro[3,2-e]pyrido[4,3-b]indol;
2,9-Dimethyl-6-allyl-6H-7,8,9,10-tetrahydrofuro[3,2-e]pyrido[4,3-b]indol;

2,9-Dimethyl-6-(3,4,5-trimethoxybenzyl)-6H-7,8,9,10-tetrahydrofuro[3,2-e]pyrido[4,3-b]indol;

2,9-Dimethyl-6-(4-(4-fluorphenyl)-4-oxobutyl)-6H-7,8,9,10-tetrahydrofuro[3,2-e]pyrido[4,3-b]indol;

2-Trifluormethyl-9-methyl-6H-7,8,9,10-tetrahydrofuro[3,2-e]pyrido[4,3-b]indol;

2-Trifluormethyl-9-ethyl-6H-7,8,9,10-tetrahydrofuro[3,2-e]pyrido[4,3-b]indol;

2-Trifluormethyl-6-ethyl-9-methyl-6H-7,8,9,10-tetrahydrofuro[3,2-e]pyrido[4,3-b]indol;

2-Trifluormethyl-6,9-diethyl-6H-7,8,9,10-tetrahydrofuro[3,2-e]pyrido[4,3-b]indol;

1,2-Dimethyl-9-acetyl-6H-7,8,9,10-tetrahydrofuro[3,2-e]-pyrido[4,3-b]indol;

1,2-Dimethyl-9-ethyl-6H-7,8,9,10-tetrahydrofuro[3,2-e]-pyrido[4,3-b]indol;

1,2-Dimethyl-9-(2,2,2-trifluorethyl)-6H-7,8,9,10-tetrahydrofuro[3,2-e]pyrido[4,3-b]indol;

1,2-Dimethyl-9-isopropyl-6H-7,8,9,10-tetrahydrofuro[3,2-e]pyrido[4,3-b]indol;

1,2,6-Trimethyl-6H-7,8,9,10-tetrahydrofuro[3,2-e]pyrido[4,3-b]indol;

1,2,6-Trimethyl-9-trifluoracetyl-6H-7,8,9,10-tetrahydrofuro[3,2-e]pyrido[4,3-b]indol;

1,2,6,9-Tetramethyl-6H-7,8,9,10-tetrahydrofuro[3,2-e]-pyrido[4,3-b]indol;

1,2,9-Trimethyl-6-acetyl-6H-7,8,9,10-tetrahydrofuro[3,2-e]pyrido[4,3-b]indol;

1,2,9-Trimethyl-6-(4-chlorbenzoyl)-6H-7,8,9,10-tetrahydrofuro[3,2-e]pyrido[4,3-b]indol;

0042103

1,2,9-Trimethyl-6-phenyl-6H-7,8,9,10-tetrahydrofuro-[3,2-e]pyrido[4,3-b]indol;
1,2,9-Trimethyl-6-(4-nitrophenyl)-6H-7,8,9,10-tetrahydrofuro[3,2-e]pyrido[4,3-b]indol;
1,2,9-Trimethyl-6-(4-aminophenyl)-6H-7,8,9,10-tetrahydrofuro[3,2-e]pyrido[4,3-b]indol;
1,2,9-Trimethyl-6-(4-acetylaminophenyl)-6H-7,8,9,10-tetrahydrofuro[3,2-e]pyrido[4,3-b]indol;
1,2,9-Trimethyl-6-(3-dimethylaminopropyl)-6H-7,8,9,10-tetrahydrofuro[3,2-e]pyrido[4,3-b]indol;
1,2,9-Trimethyl-6-(3-piperidinopropyl)-6H-7,8,9,10-tetrahydrofuro[3,2-e]pyrido[4,3-b]indol;
2-Phenyl-6H-7,8,9,10-tetrahydrofuro[3,2-e]pyrido-[4,3,-b]indol;
2-Phenyl-9-ethyl-6H-7,8,9,10-tetrahydrofuro[3,2-e]-pyrido[4,3-b]indol;
2-Phenyl-9-(4-methylpiperazinocarbonyl)-6H-7,8,9,10-tetrahydrofuro[3,2-e]pyrido[4,3-b]indol;
2-Phenyl-9-methyl-6H-7,8,9,10-tetrahydrofuro[3,2-e]-pyrido[4,3-b]indol;
2-Phenyl-9-methyl-6-(3-diethylaminopropyl)-6H-7,8,9,10-tetrahydrofuro[3,2-e]pyrido[4,3-b]indol;
1-Methyl-2-phenyl-6H-7,8,9,10-tetrahydrofuro[3,2-e]-pyrido[4,3-b]indol;
1,9-Dimethyl-2-phenyl-6H-7,8,9,10-tetrahydrofuro[3,2-e]-pyrido[4,3-b]indol.

Zur vorliegenden Erfindung gehören pharmazeutische Zubereitungen, die neben nicht-toxischen, inerten, pharmazeutisch geeigneten Trägerstoffen eine oder mehrere der erfindungsgemäßen Verbindungen oder deren Salze enthalten oder die aus einer oder mehrerer der erfindungsgemäßen Verbindungen oder deren Salzen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einen Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten, pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat,

(e) Lösungsverzögerer, z.B. Paraffin, und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit, und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett, und höhere Ester (z.B. $C_{14}$-Alkohol mit $C_{16}$-Fettsäure) oder Gemische dieser Stoffe.

Lösungen und Emulsionen können neben dem oder den Wirkstoffen die üblichen Trägerstoffe wie Lösungsmittel, Lösungsvermittler und Emulgator, z.B. Wasser, Ethylalkohol, Isopropylalkohol, Ethylcarbonat, Ethylacetat,

0042103

Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylenglykol, Suspendiermittel, z.B. ethoxylierte Isostearylalkohole, Polyoxyethylensorbit- und -sorbitanester, mikrokristalline Cellulose, Aluminiummetahydroxid, Bentonid, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen

können außer Verbindungen der Formel (I) und/oder deren Salzen auch andere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehören auch die Verwendung der Verbindungen der Formel (I) und/oder deren Salzen sowie von pharmazeutischen Zubereitungen, die eine oder mehrere Verbindungen der Formel (I) und/oder deren Salze enthalten, in der Humanmedizin zur Verhütung, Besserung und/oder Heilung der oben aufgeführten Erkrankungen.

Die Wirkstoffe oder die pharmazeutischen Zubereitungen können vorzugsweise oral, parenteral und/oder rectal, vorzugsweise oral und parenteral, insbesondere oral und intravenös appliziert werden.

Im allgemeinen hat es sich als vorteilhaft erwiesen, den oder die Wirkstoffe bei parenteraler (i.v. oder i.m.) Applikation in Mengen von etwa 0,01 bis etwa 10, vorzugsweise von 0,1 bis 1 mg/kg Körpergewicht je 24 Stunden und bei oraler Applikation in Mengen von etwa 0,05 bis etwa 100, vorzugsweise 0,1 bis 10 mg/kg Körpergewicht je 24 Stunden, gegebenenfalls in Form mehrerer Einzelgaben zur Erzielung der gewünschten Ergebnisse zu verabreichen. Eine Einzelgabe enthält den oder die Wirkstoffe, vorzugsweise in Mengen von etwa 0,01 bis etwa 30, insbesondere 0,03 bis 3 mg/kg Körpergewicht.

Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Zur vorliegenden Erfindung gehören auch solche Arzneimittel, die neben Verbindungen der Formel (I) noch weitere Wirkstoffe enthalten. Vorzugsweise seien genannt:

ß-Rezeptorenblocker, Parasympathikolytika, Anxiolytika, Neuroleptika, Hypnotika und Tranquilizer.

Beispiel 1

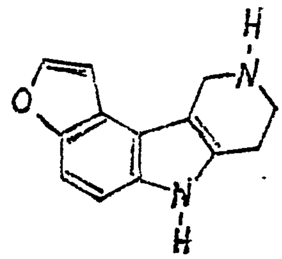

6H-7,8,9,10-tetrahydrofuro[3,2-e]pyrido[4,3-b]indol

0,022 Mol 5-Hydrazinobenzofuranhydrochlorid suspendiert man unter Rühren in 200 ml Isopropanol, versetzt mit 0,026 Mol 4-Piperidonhydrochloridmonohydrat und erhitzt 2 Stunden zum Sieden. Man dampft zur Trockene ein, alkalisiert mit 2n Natronlauge und extrahiert mit Dichlormethan. Zur Reinigung wird aus Isopropanol umkristallisiert.

Ausbeute: 70 % der Theorie; Fp. 183°C.

Beispiel 2

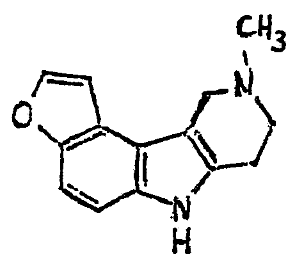

9-Methyl-6H-7,8,9,10-tetrahydrofuro[3,2-e]pyrido[4,3-b]-indol

0,1 Mol 5-Hydrazinobenzofuranhydrochlorid werden in 300 ml Isopropanol mit 0,11 Mol 1-Methyl-4-piperidon versetzt und zum Sieden erhitzt. Man fügt 100 ml HCl-gesättigtes Isopropanol hinzu und kocht 1/2 bis 1 Stunde. Nach dem Abdampfen des Lösungsmittels behandelt man den Rückstand mit wäßriger Natronlauge und extrahiert die freie Base mit Ethylacetat. Umkristallisation aus Isopropanol liefert das reine Produkt.

Ausbeute: 67 % der Theorie; Fp. 236-238 °C;
Lactat: Fp. 196 °C.

Beispiel 3

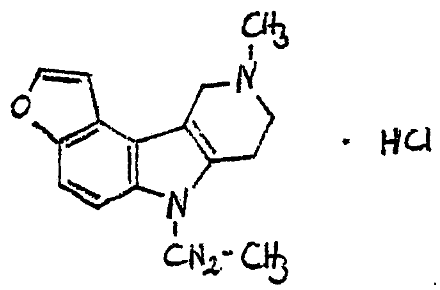

9-Methyl-6-ethyl-6H-7,8,9,10-tetrahydrofuro[3,2-e]pyrido-[4,3-b]indolhydrochlorid

Man löst 0,02 Mol 9-Methyl-6H-7,8,9,10-tetrahydrofuro-[3,2-e]pyrido[4,3-b]indol in 100 ml abs. Dimethylsulfoxid, setzt 0,02 Mol Natriumhydrid hinzu und tropft nach einstündigem Rühren 0,022 Mol Ethylbromid bei 20 °C ein. Nach einer weiteren Stunde gießt man in Eiswasser, extrahiert mit Ethylacetat und chromatographiert das Rohprodukt an Kieselgel; Elutionsmittel: Dichlormethan/Methanol (95:5).

Die ölige Base wird mit Isopropanol-HCl in das kristalline Hydrochlorid umgewandelt.

Ausbeute: 45 % der Theorie; Fp. 239 °C.

Beispiel 4

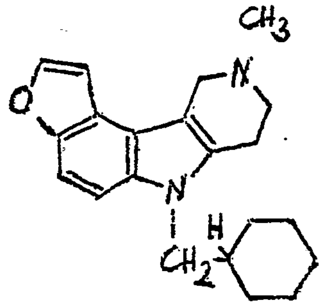

9-Methyl-6-cyclohexylmethyl-6H-7,8,9,10-tetrahydrofuro-[3,2-e]pyrido[4,3-b]indol

0,1 Mol 9-Methyl-6H-7,8,9,10-tetrahydrofuro[3,2-e]pyrido[4,3-b]indol werden in 500 ml abs. Dimethylformamid mit 0,1 Mol Natriumhydrid versetzt und nach 2 Stunden durch Zugabe von 0,11 Mol Cyclohexylmethylbromid bei 20°C zur Reaktion gebracht. Nach 16 Stunden arbeitet man in der üblichen Weise auf und kristallisiert die Rohbase aus Isopropanol um.

Ausbeute: 50 % der Theorie; Fp. 160°C.

Beispiel 5

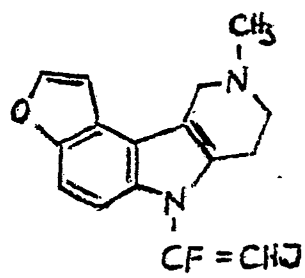

9-Methyl-6-(1-fluor-2-iodethenyl)-6H-7,8,9,10-tetra-hydrofuro[3,2-e]pyrido[4,3-b]indol

Man löst 0,05 Mol 9-Methyl-6H-7,8,9,10-tetrahydrofuro-[3,2-e]pyrido[4,3-b]indol in 250 ml abs. Dimethylformamid, gibt 0,05 Mol Natriumhydrid hinzu und rührt 2 Stunden bei 20°C. Anschließend gibt man 0,05 Mol 2-Jod-1,1,1-trifluorethan hinzu, läßt 24 Stunden bei 20°C nachreagieren und gießt dann auf Wasser. Das ausgefallene Produkt wird in Ethylacetat aufgenommen und nach dem Abdestillieren des Lösungsmittels aus Diethylether umkristallisiert.

Ausbeute: 15 % der Theorie; Fp. 147°C.

Beispiel 6

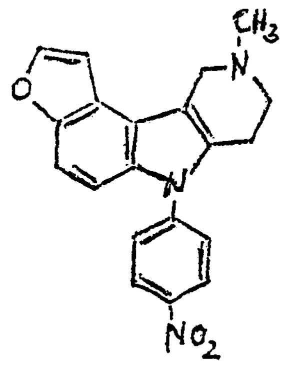

9-Methyl-6-(4-nitrophenyl)-6H-7,8,9,10-tetrahydrofuro/3,2-e7pyrido/4,3-b7indol

0,05 Mol 9-Methyl-6H-7,8,9,10-tetrahydrofuro/3,2-e7-pyrido/4,3-b7indol löst man in 250 ml abs. Dimethylformamid, fügt 0,05 Mol Natriumhydrid hinzu und läßt 2 Stunden bei 20°C rühren. Nun tropft man 0,055 Mol 4-Fluornitrobenzol ein, läßt 1 Stunde nachreagieren und gießt anschließend auf Eiswasser. Man extrahiert das Reaktionsprodukt mit Ethylacetat und kristallisiert zuletzt aus Isopropanol um.

Ausbeute: 74 % der Theorie; Fp. 189°C;
Lactat: Fp. 177°C.

Beispiel 7

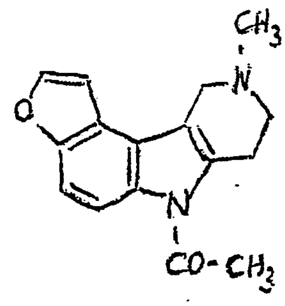

a) 9-Methyl-6-acetyl-6H-7,8,9,10-tetrahydrofuro[3,2-e]pyrido[4,3-b]indol

0,1 Mol 9-Methyl-6H-7,8,9,10-tetrahydrofuro[3,2-e]pyrido[4,3-b]indol läßt man in 500 ml abs. Dimethylformamid mit 0,1 Mol Natriumhydrid reagieren, fügt 0,11 Mol Acetylchlorid hinzu und rührt 2 Stunden bei 20°C.

Die säulenchromatographische Reinigung an Kieselgel (Elutionsmittel: Aceton) liefert fast reines Produkt, das aus Isopropanol rekristallisiert wird.

Ausbeute: 20 % der Theorie; Fp. 148°C.

b) 0,1 Mol 9-Methyl-6H-7,8,9,10-tetrahydrofuro[3,2-e]pyrido[4,3-b]indol läßt man in 500 ml abs. Dimethylformamid in Gegenwart von 0,1 Mol N,N-Dimethylanilin mit 0,1 Mol Acetanhydrid 2 Stunden bei 20°C reagieren. Nach dem Eingießen in Eiswasser extrahiert man das Produkt mit Ethylacetat und reinigt durch Umkristallisation aus Isopropanol.

Ausbeute: 25 % der Theorie; Fp. 148°C.

TP 35

Beispiel 8

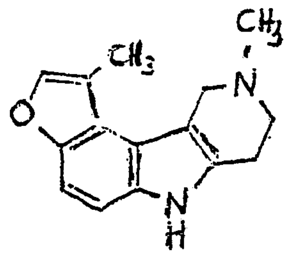

1,9-Dimethyl-6H-7,8,9,10-tetrahydrofuro/3,2-e7pyrido-/4,3-b7indol

0,3 Mol 3-Methyl-5-hydrazinobenzofuran und 0,32 Mol 1-Methyl-4-piperidon werden in 250 ml Ethylenglykol 2 Stunden auf 180 bis 190°C erhitzt. Man läßt erkalten, gießt in Eiswasser und filtriert die Kristallfällung ab. Das getrocknete Produkt wird in Toluol gelöst und an Aluminiumoxid (neutral) chromatographiert. Nach dem Eluieren mit Toluol/Methanol (10:1) wird die Verbindung aus Isopropanol umkristallisiert.

Ausbeute: 35 % der Theorie; Fp. 183-184°C;
Lactat: Fp. 205°C.

Beispiel 9

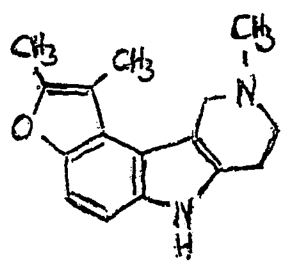

1,2,9-Trimethyl-6H-7,8,9,10-tetrahydrofuro/3,2-e7pyrido/4,3-b7indol

0,23 Mol 2,3-Dimethyl-5-hydrazinobenzofuran und 0,25

Mol 1-Methyl-4-piperidon werden in 350 ml Ethylenglykol unter $N_2$-Schutzgasatmosphäre 1 Stunde auf 180 bis 190°C erhitzt. Die beim Erkalten ausfallenden Kristalle werden abfiltriert, mit wenig Wasser und Isopropanol gewaschen und getrocknet. Zur weiteren Reinigung löst man in Aceton und chromatographiert an neutralem Aluminiumoxid mit Aceton. Die vereinigten Eluate werden eingedampft, der Rückstand aus Ethylacetat rekristallisiert.

Ausbeute: 51 % der Theorie; hellgelbe Kristalle, Fp. 223-224°C (Zers);
Lactat: farblose Kristalle vom Fp. 216-217°C.

Beispiel 10

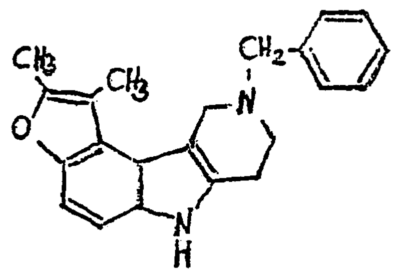

9-Benzyl-1,2-dimethyl-6H-7,8,9,10-tetrahydrofuro /3,2-e/-pyrido/4,3-b/indol

a) 0,3 Mol 2,3-Dimethyl-5-hydrazinobenzofuran und 0,33 Mol 1-Benzyl-4-piperidon werden unter Rühren und $N_2$-Schutzgasatmosphäre in 100 ml Ethylenglykol suspendiert und langsam auf 180°C erhitzt. Nach 1 Stunde läßt man auf 20°C abkühlen, wobei sich das Produkt ölig ausscheidet. Man dekantiert ab, nimmt das Öl in Toluol auf und reinigt säulenchromatographisch an neutralem Aluminiumoxid (Elutionsmittel: Toluol/

Isopropanol (100:2)). Aus den eingedampften Eluaten erhält man gelbbraunes Produkt, das aus Isopropanol rekristallisiert wird.

Ausbeute: 42 % der Theorie; Fp. 178°C.

b) 0,2 Mol 2,3-Dimethy-5-hydrazinobenzofuranhydrochlorid und 0,25 Mol 1-Benzyl-4-piperidon löst man in der Kälte in 500 ml Ethanol. Die Lösung wird zum Sieden gebracht und in der Hitze mit 150 ml HCl-gesättigtem Isopropanol versetzt. Nach 3-stündigem Kochen wird das Lösungsmittel abdestilliert, der Rückstand mit Natriumhydroxid behandelt und in Methylenchlorid aufgenommen. Die getrocknete Methylenchloridlösung wird auf eine neutrale Aluminiumoxid-Säule gegeben und mit Toluol/Methanol (100:2) eluiert.

Ausbeute: 36 % der Theorie; Fp. 178°C nach Umkristallisation aus Isopropanol;
Lactat: Fp. 165°C.

Beispiel 11

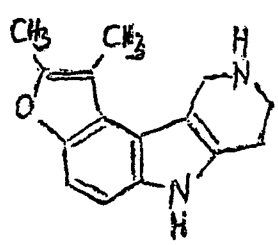

1,2-Dimethyl-6H-7,8,9,10-tetrahydrofuro/3,2-e/pyrido/4,3-b/indol

0,1 Mol 9-Benzyl-1,2-dimethyl-6H-7,8,9,10-tetrahydrofuro/3,2-e/pyrido/4,3-b/indol werden in 600 ml Methanol

in Gegenwart von 3,0 g Palladiumkohle (5 %ig) bei 40°C bis zur Aufnahme von 0,1 Mol Wasserstoff hydriert.

Ausbeute: 95 % der Theorie; Fp. 215°C (Zers.);
Lactat: Fp. 206-207°C.

Beispiel 12

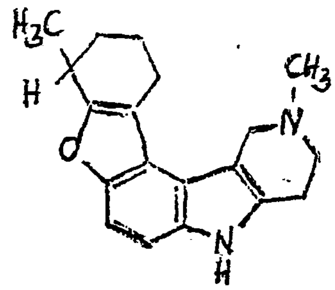

4,11-Dimethyl-8H-9,10,11,12-tetrahydropyrido[3,4-b]cyclohexan[4,5]furo[3,2-e]indol

0,18 Mol 2-Hydrazino-6-methyl-6,7,8,9-tetrahydrodibenzofuran werden in 300 ml Ethylenglykol mit 0,20 Mol 1-Methyl-4-piperidon unter Rühren eine Stunde auf 180 bis 190°C erhitzt. Nach beendeter $NH_3$-Entwicklung läßt man auf Raumtemperatur abkühlen, saugt den Kristallbrei ab und wäscht mit wenig Isopropanol nach. Zur Reinigung wird aus Ethylacetat umkristallisiert.

Ausbeute: 62 % der Theorie; Fp. 191 bis 192°C.
Lactat: Fp. 202 - 203°C.

Beispiel 13

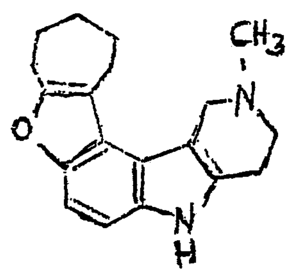

TP 35

12-Methyl-1,2,3,4,5,10,11,12,13-nonahydro-9H-pyrido-[3,4-b]cycloheptan[4,5]furo[3,2-e]indol

0,12 Mol 2-Hydrazino-7,8,9,10-tetrahydro-6H-benzo[b]-cyclohepta[d]furan und 0,13 Mol 1-Methyl-4-piperidon werden in 250 ml Ethylenglykol 2 Stunden auf 180 bis 185°C erhitzt. Man läßt auf 80°C abkühlen, tropft unter Rühren 100 ml Wasser hinzu und saugt den sich ausscheidenden Kristallbrei ab. Die Reinigung des Produktes erfolgt durch Chromatographie an Kieselgel mit Methylenchlorid/Methanol (6:4) mit anschließender Rekristallisation aus Ethylacetat.

Ausbeute: 55 % der Theorie; Fp. 206 bis 207°C;
Lactat: Fp. 209-211°C.

Beispiel 14

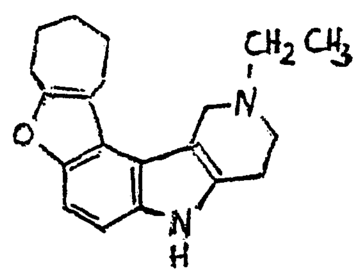

12-Ethyl-1,2,3,4,5,10,11,12,13-nonahydro-9H-pyrido[3,4-b]cycloheptan[4,5]furo[3,2-e]indol

0,12 Mol 2-Hydrazino-7,8,9,10-tetrahydro-6H-benzo[b]-cyclohepta[d]furan und 0,13 Mol 1-Ethyl-4-piperidon werden wie in Beispiel 13 beschrieben umgesetzt und aufgearbeitet.

Ausbeute: 40 % der Theorie; Fp. 180 bis 181°C;
Lactat: 200 bis 201°C.

TP 35

Beispiel 15

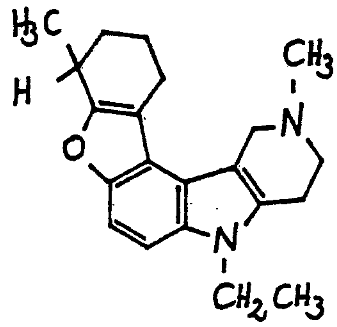

4,11-Dimethyl-8-ethyl-8H-9,10,11,12-tetrahydropyrido[3,4-b]-cyclohexan[4,5]furo[3,2-e]indol

Die Verbindung entsteht analog der in Beispiel 3 beschriebenen aus 0,02 Mol 4,11-Dimethyl-8H-9,10,11,12-tetrahydropyrido[3,4-b]cyclohexan-[4,5]furo[3,2-e]indol, 0,02 Mol Natriumhydrid und 0,022 Mol Ethylbromid in Dimethylformamid bei 20 °C.

Ausbeute: 48 % der Theorie; Fp. 146 °C (aus Ethanol).

Beispiel 16

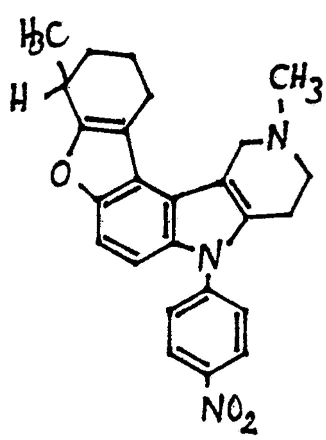

4,11-Dimethyl-8-(4-nitrophenyl)-8H-9,10,11,12-tetrahydropyrido[3,4-b]-cyclohexan[4,5]furo[3,2-e]indol

Die Verbindung entsteht analog Beispiel 6 aus 0,1 Mol 4,11-Dimethyl-8H-9,10,11,12-tetrahydropyrido[3,4-b]cyclohexan[4,5]furo[3,2-e]indol, 0,1 Mol Natriumhydrid und 0,1 Mol 4-Fluornitrobenzol in Dimethylformamid bei 20 °C.

Ausbeute: 53 % der Theorie; Fp. 196-197 °C (aus Ethanol).

Hydrochlorid: Fp. 291-293 °C (Zers.).

Beispiel 17

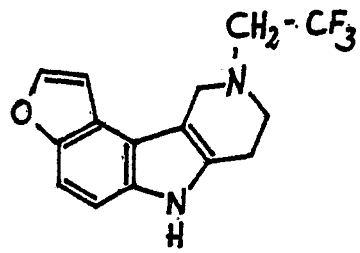

9-(2,2,2-Trifluorethyl)-6H-7,8,9,10-tetrahydrofuro[3,2-e]pyrido-[4,3-b]indol

0,02 Mol 5-Hydrazinobenzofuran und 0,022 Mol 1-(2,2,2-Trifluorethyl)-4-piperidon suspendiert man unter Rühren in 20 ml Ethylenglykol und erhitzt 30 Minuten auf 190 °C. Nach dem Abkühlen versetzt man mit Wasser, extrahiert mit Diisopropylether, reinigt säulenchromatographisch an neutralem Aluminiumoxid (Elutionsmittel: Dichlormethan) und rekristallisiert aus Diisopropylether/Petrolether (40-60 °).

Ausbeute: 38 % der Theorie; Fp. 110-112 °C.

TP 35

Beispiel 18

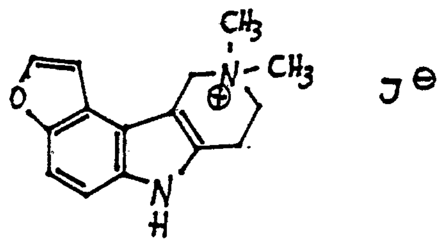

9-Methyl-6H-7,8,9,10-tetrahydrofuro[3,2-e]pyrido[4,3-b]indol-$N^{(9)}$-methoiodid

0,1 Mol 9-Methyl-6H-7,8,9,10-tetrahydrofuro[3,2-e]pyrido[4,3-b]indol werden in 1200 ml Methanol gelöst, mit 50 ml Iodmethan versetzt und 24 Stunden bei Raumtemperatur stehengelassen. Nach dem Einengen auf das halbe Volumen kristallisiert das Methoiodid in reiner Form aus.

Ausbeute: 84 % der Theorie; Fp. 257 °C (Zers.).

TP 35

| Beispiel | Ausgangsverbindung (II) 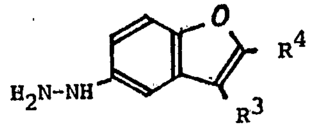 | | Ausgangsverbindung (III) 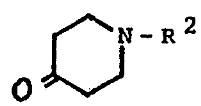 | Endverbindung (I) 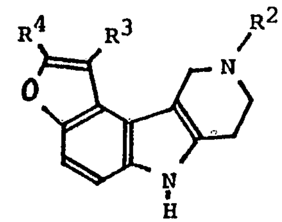 | | Versuchsbedingungen |
|---|---|---|---|---|---|---|
| | $R^3$ | $R^4$ | $R^2$ | Schmelzpunkt (°C) | Ausb. Base % d.Theorie | Reaktionsmedium<br>Reaktionsdauer<br>Temperatur |
| 19 | -H | 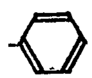 | $-CH_3$ | 222 (Base) | 70 | (a) Isopropanol/HCl; 30 Min.; Rückflußtemperatur.<br>(b) Ethylenglykol; 60 Min. 190 °C. |
| 20 | $-CH_3$ | $-CH_3$ | $-CH_2CF_3$ | 184-187 (Base) | 36 | Ethylenglykol; 60 Min.; 190 °C |
| 21 | $-CH_3$ | 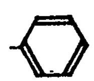 | $-CH_3$ | 205-206 (Base) | 45 | Ethylenglykol; 35 Min.; 190 °C |
| 22 | -H | -H | $-COOCH_2CH_3$ | 211-214 | 77 | Isopropanol/HCl; 30 Min.; Rückflußtemperatur |

TP 35

| Beispiel | Ausgangsverbindung 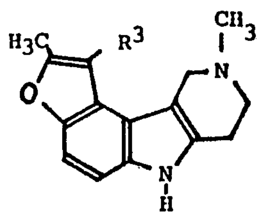 | Endverbindung (I) 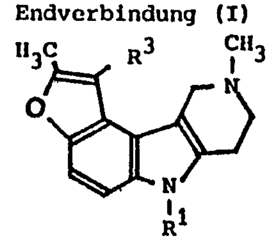 | | | Versuchsbedingungen<br>Reaktionsmedium<br>Alkylierungs- bzw. Acylierungsmittel<br>Reaktionsdauer<br>Temperatur |
|---|---|---|---|---|---|
| | $R^3$ | $R^1$ | Schmelzpunkt (°C) | Ausb. Base % d. Theorie | |
| 23 | -H | 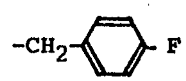 | 154-155 (Base)<br>298-300 (HCl) | 35 | Dimethylformamid/NaH; Cl-$R^1$;<br>30 Min. bei -5 °C, dann 16 Stdn. bei -30 °C |
| 24 | -H | 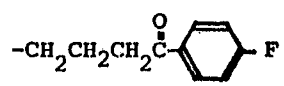 | 122-123 (Base)<br>139-140 (Lactat) | 65 | Dimethylformamid/NaH; Cl-$R^1$ als Ethylenketal; 16 Std.; 20 °C;<br>Entketalisierung in HCOOH b. 20 °C |
| 25 | $-CH_3$ | 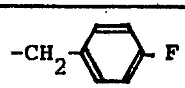 | 126 (Base) | 30 | Dimethylformamid/NaH; Cl-$R^1$;<br>60 Min.; 20 °C |
| 26 | $-CH_3$ | 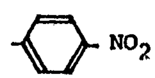 | 205 (Z.) (Base) | 62 | Dimethylformamid/NaH; F-$R^1$ (p);<br>90 Min.; 20 °C |
| 27 | $-CH_3$ | 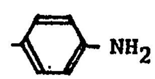 | > 250 (Z.)<br>(Dihydrochlorid) | 85<br>(2 HCl) | Hydrierung von Beisp. 26 in $CH_3OH$/<br>Pd-C; Fällung mit Ether/HCl |
| 28 | $-CH_3$ | 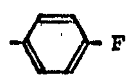 | 219-222 (Base) | 30 | N-Methylpyrrolidon in Gegenwart v. $Cu_2Br_2$ u. $Na_2CO_3$; $R^1$-Br; 3 Stdn.;<br>190-200 °C |
| 29 | $-CH_3$ | 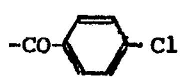 | 155 (Base) | 28 | Dimethylformamid/NaH; Cl-$R^1$;<br>120 Min.; -20 bis 20 °C ansteig. |

TP 35

| Beispiel | Ausgangsverbindung 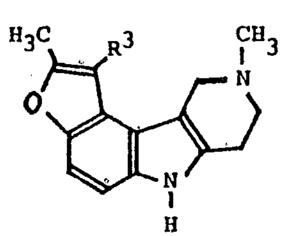 | Endverbindung (I) 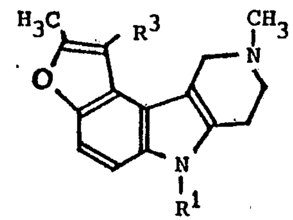 | | | Versuchsbedingungen<br>Reaktionsmedium<br>Alkylierungsmittel bzw.<br>Acylierungsmittel<br>Reaktionsdauer<br>Temperatur |
|---|---|---|---|---|---|
| | $R^3$ | $R^1$ | Schmelzpunkt (°C) | Ausb. Base % d.Theorie | |
| 30 | $-CH_3$ | $-CO-CH_2CH_3$ | 155 (Base) | 36 | Dimethylformamid/NaH; $ClCOCH_2CH_3$<br>180 Min.; -20 bis 20 °C |
| 31 | $-CH_3$ | $-CH_3$ | 160-161 (Base) | 57 | Dimethylformamid/NaH; $ICH_3$;<br>90 Min.; 20 °C |
| 32 | $-CH_3$ | $-CH_2CH_3$ | 221-222 (Base) | 38 | Dimethylformamid/NaH; $BrCH_2CH_3$;<br>30 min.; 4 °C |
| 33 | $-CH_3$ | $-CH_2CH_2CH_2-N(CH_3)_2$ | > 260 (Z.)<br>(Dihydrochlorid) | 52<br>(2 HCl) | Dimethylformamid/NaH; $Cl-R^1$;<br>120 Min.; 0 ° bis 20 °C |

0042103

Patentansprüche

1. 7,8,9,10-Tetrahydrofuro[3,2-e]pyrido[4,3-b]indol-Derivate der allgemeinen Formel I

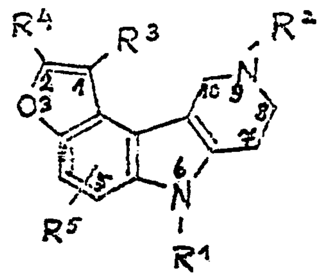

(I)

in welcher

$R^1$ und $R^2$ gleich oder verschieden sind und jeweils für Wasserstoff, geradkettiges, verzweigtes oder cyclisches Alkyl, Alkenyl, Alkinyl, Aryl, Acyl, Aroyl, Mono- und Dialkylaminoalkyl, Arylalkylaminoalkyl, Heteroarylalkylaminoalkyl oder Acylamino stehen, wobei die genannten Alkyl-, Alkenyl- und Arylreste gegebenenfalls substituiert sind durch Halogen, Nitro, Amino, Alkyl, Trifluormethyl, Alkoxy, Hydroxy, Acylamino, gegebenenfalls substituiertes Phenyl, Heteroaryl oder durch einen 5-, 6- oder 7-gliedrigen gesättigten oder ungesättigten aliphatischen Ring, der 1 oder 2 gleiche oder verschiedene Heteroatome aus der Gruppe Sauerstoff, Schwefel oder N-R' enthalten kann, wobei

R' für Wasserstoff, Alkyl, Alkenyl, Aryl, Aralkyl, Acyl oder Aroyl steht,

$R^3$ und $R^4$ gleich oder verschieden sind und jeweils für Wasserstoff, geradkettiges, verzweigtes oder cyclisches Alkyl, Alkenyl oder Alkinyl, Aryl, Acyl oder Aroyl stehen, wobei die genannten Alkyl-, Alkenyl- und Arylreste gegebenenfalls substituiert sind

durch Halogen, Alkyl, Alkoxy, Acylamino, gegebenenfalls substituiertes Phenyl oder Heteroaryl, wobei $R^3$ nicht für Wasserstoff steht, wenn $R^2$ und $R^4$ Methyl bedeuten und $R^1$ Wasserstoff bedeutet, oder

$R^3$ und $R^4$ gemeinsam für einen gegebenenfalls verzweigten Alkylenrest stehen, durch Sauerstoff oder durch die Gruppe $S(O)_n$ unterbrochen sein kann, wobei n für 0, 1 oder 2 steht,

$R^5$ für Wasserstoff, Halogen, Alkyl, Alkoxy oder Phenyl steht,

als solche, in Form ihrer quarternären Ammoniumsalze gebildet mit Alkylhalogeniden und ihre pharmakologisch unbedenklichen Säureadditionssalze.

2. Verbindung der allgemeinen Formel I gemäß Anspruch 1, in welcher

$R^1$ und $R^2$ gleich oder verschieden sind und jeweils für Wasserstoff, geradkettiges, verzweigtes oder cyclisches Alkyl, Alkenyl oder Alkinyl mit bis zu 12 Kohlenstoffatomen, Aralkyl mit 7 bis 16 Kohlenstoffatomen, Phenyl, Acyl mit bis zu 8 Kohlenstoffatomen, Benzoyl, Mono- und Dialkylaminoalkyl und Phenylalkylaminoalkyl mit jeweils bis zu 4 Kohlenstoffatomen in den Alkylresten, oder Acylamino. dessen Stickstoffatom substituiert sein kann durch 1 oder 2 Alkylreste mit 1 bis 4 Kohlenstoffatomen oder durch Phenyl, wobei die beiden Alkylgruppen gegebenenfalls mit dem Stickstoffatom einen 5- bis 7-gliedrigen Ring bilden, der gegebenenfalls ein weiteres Heteroatom aus der Gruppe Sauerstoff, NH oder N-Alkyl mit 1-4 Kohlenstoffatomen enthält, stehen,

wobei die vorgenannten Alkyl-, Alkenyl- und Phenylreste ihrerseits gegebenenfalls substituiert sind durch Halogen, Nitro, Amino, Hydroxy, Alkyl mit 1 bis 4 Kohlenstoffatomen, Alkoxy mit 1 bis 4 Kohlenstoffatomen, Acylamino mit 1 bis 4 Kohlenstoffatomen in der Acylgruppe, Phenyl, Pyridyl oder durch einen 5-, 6- oder 7-gliedrigen gesättigten oder ungesättigten aliphatischen Ring, der 1 oder 2 gleiche oder verschiedene Heteroatome aus der Gruppe Sauerstoff, Schwefel oder NR' enthalten kann, wobei

R' für Wasserstoff, Alkyl mit 1 bis 4 Kohlenstoffatomen, Phenyl, Benzyl oder Acyl mit bis zu 4 Kohlenstoffatomen steht,

$R^3$ und $R^4$ gleich oder verschieden sind und jeweils für Wasserstoff, geradkettiges, verzweigtes oder cyclisches Alkyl, Alkenyl oder Alkinyl mit bis zu 12 Kohlenstoffatomen, Aryl mit 6 bis 12 Kohlenstoffatomen, Acyl mit bis zu 4 Kohlenstoffatomen oder Benzoyl stehen, wobei die vorgenannten Alkyl-, Alkenyl- und Arylreste gegebenenfalls substituiert sind durch Halogen, Trifluormethyl, Alkyl mit 1 bis 4 Kohlenstoffatomen, Alkoxy mit 1 bis 4 Kohlenstoffatomen, Acylamino mit bis zu 4 Kohlenstoffatomen, Phenyl oder Pyridyl, wobei der Phenylrest gegebenenfalls 1 oder 2 gleiche oder verschiedene Substituenten aus der Gruppe Halogen, Trifluormethyl, Alkyl oder Alkoxy mit jeweils 1 bis 2 Kohlenstoffatomen substituiert ist,

wobei $R^3$ nicht Wasserstoff bedeutet, wenn $R^2$ und $R^4$ Methyl bedeuten und $R^1$ Wasserstoff bedeutet,

oder

$R^3$ und $R^4$ gemeinsam für einen gegebenenfalls verzweigten Alkylenrest mit bis zu 10 Kohlenstoffatomen stehen, der durch Sauerstoff, Schwefel oder die Gruppe $SO_2$ unterbrochen sein kann, und

$R^5$ für Wasserstoff, Halogen, Phenyl, Alkyl oder Alkoxy mit je 1 bis 4 Kohlenstoffatomen steht.

TP 35

3. Verbindungen der allgemeinen Formel I gemäß Anspruch 1, in welcher

$R^1$ und $R^2$ gleich oder verschieden sind und jeweils für Wasserstoff, geradkettiges, verzweigtes oder cyclisches Alkyl oder Alkenyl mit bis zu 8 Kohlenstoffatomen, Phenyl, Acyl mit bis zu 4 Kohlenstoffatomen, Benzoyl oder Acylamino stehen, wobei der Stickstoff des Aminorestes gegebenenfalls durch 1 oder 2 Alkylgruppen substituiert ist oder ein Ringglied eines Morpholin-, Piperidin- oder Piperazinrestes darstellt, deren Ringe ihrerseits gegebenenfalls durch Methyl oder Ethyl substituiert sind, wobei die vorgenannten Alkyl-, Alkenyl-, Phenyl- und Acylreste ihrerseits gegebenenfalls substituiert sind durch Alkyl mit 1 bis 4 Kohlenstoffatomen, Mono- oder Dialkylamino mit jeweils 1 bis 4 Kohlenstoffatomen im Alkylrest, Halogen, Nitro, Amino, Trifluormethyl, Trifluormethoxy, Alkoxy mit 1 bis 2 Kohlenstoffatomen im Alkoxyrest, Phenyl, Piperidin, Acetylamino, Morpholino oder Cyano,

$R^3$ und $R^4$ gleich oder verschieden sind und jeweils für Wasserstoff, Alkyl mit 1 bis 4 Kohlenstoffatomen, Phenyl oder Trifluormethyl stehen, wobei die genannten Phenyl- und Alkylreste ihrerseits gegebenenfalls substituiert sind durch Halogen, Alkyl oder Alkoxy mit jeweils 1 bis 2 Kohlenstoffatomen, wobei $R^3$ nicht für Wasserstoff steht, wenn $R^2$ und $R^4$ Methyl bedeuten und $R^1$ Wasserstoff bedeutet,

oder

$R^3$ und $R^4$ gemeinsam für einen gegebenenfalls verzweigten Alkylenrest mit bis zu 6 Kohlenstoffatomen stehen, der durch Sauerstoff oder Schwefel in der Kette unterbrochen sein kann und

$R^5$ für Wasserstoff, Halogen oder Methyl steht.

4. Verfahren zur Herstellung von Verbindungen der allgemeinen Formel I gemäß Anspruch 1, dadurch gekennzeichnet, daß man Hydrazinverbindungen der allgemeinen Formel II

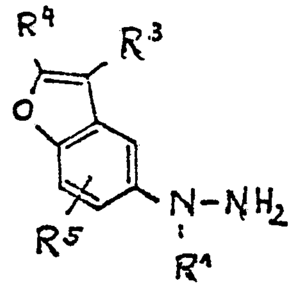

(II)

in welcher

$R^1$, $R^3$, $R^4$ und $R^5$ die oben angegebene Bedeutung haben,

mit Piperidonen der allgemeinen Formel (III)

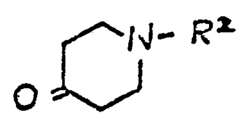

(III)

in welcher

$R^2$ die oben angegebene Bedeutung hat,

bzw. mit Salzen dieser Piperidone mit anorganischen oder organischen Säuren in Gegenwart von inerten organischen Lösungsmitteln bei Temperaturen zwischen 20 und 250°C, gegebenenfalls in Gegenwart von Kondensationsmitteln, umsetzt und die so erhaltenen Verbindungen der Formel (I) gegebenenfalls anschließend in bekannter Weise in die quaternären Ammoniumsalze oder die Säureadditionssalze überführt und für den Fall, daß die Stickstoffsubstituenten $R^1$ und $R^2$ Wasserstoff bedeuten, diese nach bekannten Methoden durch andere erfindungsgemäße Substituenten ersetzt.

5. Verbindungen der allgemeinen Formel I gemäß Anspruch 1 zur Verwendung bei der Bekämpfung von Erkrankungen.

6. Verbindungen der allgemeinen Formel I gemäß Anspruch 1 bei der Bekämpfung von Erkrankungen des zentralen Nervensystems.

7. Arzneimittel enthaltend mindestens eine Verbindung der allgemeinen Formel I gemäß Anspruch 1.

8. Verfahren zur Herstellung von Arzneimitteln, dadurch gekennzeichnet, daß man Verbindungen der allgemeinen Formel I gemäß Anspruch 1 gegebenenfalls unter Verwendung von üblichen Hilfs- und Trägerstoffen in eine geeignete Applikationsform überführt.

9. Verwendung von Verbindungen der allgemeinen Formel I gemäß Anspruch 1 bei der Bekämpfung von Erkrankungen.

10. Verwendung gemäß Anspruch 9 bei der Bekämpfung von Erkrankungen des zentralen Nervensystems.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0042103

Nummer der Anmeldung

EP 81104258.9

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | DE - A - 1 645 993 (ABBOTT)<br>* Seite 2, 2. Absatz; Beispiele 7,8 *<br>---- | 1,4,7 | C 07 D 491/147<br>A 61 K 31/44//<br>(C 07 D 491/147<br>C 07 D 209/00<br>C 07 D 307/00) |
| | | | RECHERCHIERTE SACHGEBIETE (Int. Cl.³) |
| | | | C 07 D 491/00<br>C 07 D 471/00<br>A 61 K 31/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-8

Unvollständig recherchierte Patentansprüche: -

Nicht recherchierte Patentansprüche: 9,10

Grund für die Beschränkung der Recherche:

Verfahren zur therapeutischen Behandlung des menschlichen oder tierischen Körpers, Artikel 52(4) EPÜ

**KATEGORIE DER GENANNTEN DOKUMENTE**

- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

| Recherchenort | Abschlußdatum der Recherche | Prüfer |
|---|---|---|
| WIEN | 31-08-1981 | HOCHHAUSER |